# EUROPEAN PATENT SPECIFICATION

(11) **EP 3 223 681 B1**
(45) Date of publication and mention of the grant of the patent: **09.01.2019**
(21) Application number: 15794931.4
(22) Date of filing: 17.11.2015
(51) Int. Cl.: A61B 5/00, A61B 5/021, A61B 5/024, A61B 5/026, A61B 5/0295

(54) **CPR ASSISTANCE SYSTEM AND CPR MONITORING METHOD**
HLW-UNTERSTÜTZUNGSSYSTEM UND HLW-ÜBERWACHUNGSVERFAHREN
SYSTÈME D'ASSISTANCE À LA RÉANIMATION CARDIO-RESPIRATOIRE (RCR) ET PROCÉDÉ DE SURVEILLANCE DE LA RCR

(30) Priority: 25.11.2014 EP 14194704
(43) Date of publication of application: 04.10.2017
(73) Proprietor: Koninklijke Philips N.V., 5656 AE Eindhoven (NL)
(72) Inventor: AELEN, Paul, 5656 AE Eindhoven (NL)
(74) Representative: de Haan, Poul Erik
(86) International application number: PCT/EP2015/076735
(87) International publication number: WO 2016/083183

(56) References cited:
- WO-A1-2011/154499
- KR-A- 20140 119 955
- US-A- 4 237 872
- US-A1- 2004 215 244
- US-A1- 2007 060 785
- US-A1- 2012 330 200
- US-A1- 2014 039 291

## Description

### FIELD OF THE INVENTION

This invention relates to cardiopulmonary resuscitation (CPR) and is defined by the appended claims. All other embodiments are merely exemplary.

### BACKGROUND OF THE INVENTION

Cardiopulmonary resuscitation is the procedure of giving external chest compressions and artificial ventilations to a patient that has a cardiac arrest (CA), i.e. a patient with no spontaneous heartbeat. CPR is performed in order to generate an artificial circulation and oxygenation. The compressions have to be repeated in a fast way in order to generate a sufficient blood flow to vital organs such as the heart and brain.

Cardiopulmonary resuscitation guidelines advise to do chest compressions at a compression frequency of at least 100 compressions per minute. However, these guidelines are based on statistics obtained from groups of people and do not take into account the presence of an individual optimum compression frequency. This individual optimum is a trade-off between pumping blood out of the heart and return of blood to the heart. In order to have a sustained artificial circulation, the amount of blood that is pumped out of the heart must return to the heart at every chest compression. The return of blood to the heart is called the venous return. When more blood is pumped out of the heart than is returned, a shift of blood volume can occur (e.g. blood shift to the abdominal region). When blood is shifted to other areas than the thorax (heart), it is no longer part of the circulation, and chest compressions are performed on an empty or partially empty heart, which does not generate any blood flow.

The optimal compression frequency is the frequency that results in a compression starting just after venous return has completed, i.e. after the blood flow into the right atrium of the heart is completed. A 100 cpm rate is most likely not the optimal compression rate for a lot of patients as it does not seek to enable the completion of venous return nor is it based on the physiology of a particular patient.

Document US 2014/039291 A1 relates to a medical device that comprises a control circuit configured to generate a metric indicative of a quality of cardio pulmonary resuscitation being performed on a subject and issue a recommendation to modify the cardio pulmonary resuscitation being performed on the subject based on the metric.

### SUMMARY OF THE INVENTION

The invention is defined by the claims.

According to examples in accordance with an aspect of the invention, there is provided a CPR assistance system, comprising:
a sensor for making a physiological measurement to generate a sensor signal which conveys information concerning the point in time at which venous return is completed; and
a processor for processing the sensor signal to determine time points at which CPR compression cycles should be administered based on the point in time at which venous return is completed; and
an output device providing output information relating to the determined time points.

This system is able to ensure maximum venous return by measuring physiological information which has a dependency on the venous return. The output information can be used to tune the CPR compression frequency either manually (by giving prompts to a caregiver) or automatically (by controlling an automatic CPR system). In this way, optimal patient-specific CPR frequency can be obtained with an as high as possible blood flow for keeping a sustained circulation over an extended period of time. This increases survival chances following cardiac arrest.

The sensor signal may convey information which indicates when the flow returns to zero (or near zero) after a CPR compression cycle. This can be derived from flow information or from another signal which can be considered to be a surrogate flow signal. Examples of such a surrogate flow signal are a zero derivative of pressure, or zero derivative of volume.

In one example, the sensor comprises a blood flow sensor. During the time that blood is flowing, the heart (right atrium) is being filled. This filling is ended at the time the blood flow is zero. A zero blood flow may therefore be used to indicate completion of venous return. Depending on the sensor location, a (fixed) time delay can be added to indicate completion. This time delay depends on the sensor location and should cancel out the physical delay between the sensor location and the right atrium. Alternative blood flow features (e.g. maxima/minima or level crossings) in combination with or without a delay may be used to indicate the end of venous return.

The blood flow sensor may for example comprise an external ultrasound flow sensor.

In other examples the sensor comprises a pressure sensor for measuring the (right atrial) blood pressure or a plethysmographic (volume, PG) sensor. These may also be used in a more indirect way to derive flow information. A PG sensor can be implemented by use of photoplethysmography (PPG). The time point from where the PG/pressure or derivative of the PG/pressure signal remains within a particular range for a certain time period is then representative of the end of the venous return. The pressure and PG signals can thus be used as surrogate measures for blood flow. Steady values of these signals (i.e. zero derivatives) indicate a zero flow and hence the end of venous return.

Thus, in general the processor may be adapted to determine the time at which the physiological signal remains between an upper and lower limit for a certain amount of time or the time at which the derivative of the physiological signal remains between an upper and lower limit for a certain amount of time.

Alternatively, the processor may be adapted to determine the time at which the physiological signal has its maximum value after the completion of a chest compression.

The way time points are determined will depend on the physiological parameter being measured. Furthermore, the monitoring of multiple parameters may be combined in the system, and multiple approaches for determining the time points may be combined.

The processor may for example be adapted to apply a delay to the sensed completion of the venous return to compensate for a delay between the physical location of the sensor and the actual completion of venous return at the right side of the heart.

This delay may be obtained by measuring the delay between the start of a CPR compression cycle and the start of the response of the physiological measurement sensor signal to that CPR compression cycle.

The system may be used in an emergency care monitor wherein the processor is adapted to provide an output warning signal when a CPR compression is started more than a predetermined time before and/or after the determined time points at which CPR compression cycles should be administered; or to provide an indication of a suitable compression rate corresponding to the determined time points or previously determined time points.

This provides warnings when the administered CPR is not optimum or else advises the care giver of the most suitable compression frequency. An audio and/or visual output may also or instead be provided at the timing instants which are determined as optimal for CPR compression cycles to begin. The processor may be adapted to provide an optimum compression rate, based on previously determined time points.

The system may be used in an automated CPR (ACPR) system which comprises a compression depth application system (such as a piston based ACPR device or a belt around the patient's chest). The CPR assistance system is then used for controlling the timing of operation of the compression depth application system.

Another aspect provides a CPR monitoring method, comprising:
making a physiological measurement to generate a sensor signal which conveys information concerning the point in time at which venous return is completed following a CPR compression;
processing the sensor signal to determine time points at which CPR compression cycles should be administered based on the point in time at which venous return is completed; and
providing output information relating to the determined time points.

This method may be used to advise a caregiver in which case the output information can be an audible or visible message. Alternatively, the method may be used to control an automated system in which case the output information can be control signals for controlling the compression cycles of an automated CPR system.

Making a physiological measurement may comprise making a blood flow measurement, a right atrial pressure measurement or a plethysmographic measurement.

### BRIEF DESCRIPTION OF THE DRAWINGS

Examples of the invention will now be described in detail with reference to the accompanying drawings, in which:
Figure 1 shows the flows induced by CPR;
Figure 2 shows a CPR compression pulse;
Figure 3 shows a first example of various pressure and flow signals for a spontaneous heartbeat within a patient (animal) as well as various pressure and flow signals within a patient (animal) undergoing CPR ;
Figure 4 shows a second example of various pressure and flow signals for a patient (animal) undergoing CPR;
Figure 5 is used to show how some physiological signals can be used to identify the end of the venous flow;
Figure 6 shows the method in accordance with an example;
Figure 7 shows a first example of system in accordance with an aspect of the invention; and
Figure 8 shows a second example of system in accordance with an aspect of the invention.

### DETAILED DESCRIPTION OF THE EMBODIMENTS

The invention provides a CPR assistance system, comprising a sensor for making a physiological measurement to generate a sensor signal which conveys information concerning the point in time at which venous return is completed. Time points are then determined at which CPR compression cycles should be initiated, and output information is provided related to the determined time points. The output information may be visual or audible advice to a person giving CPR or it may be control information for controlling an automated CPR system.

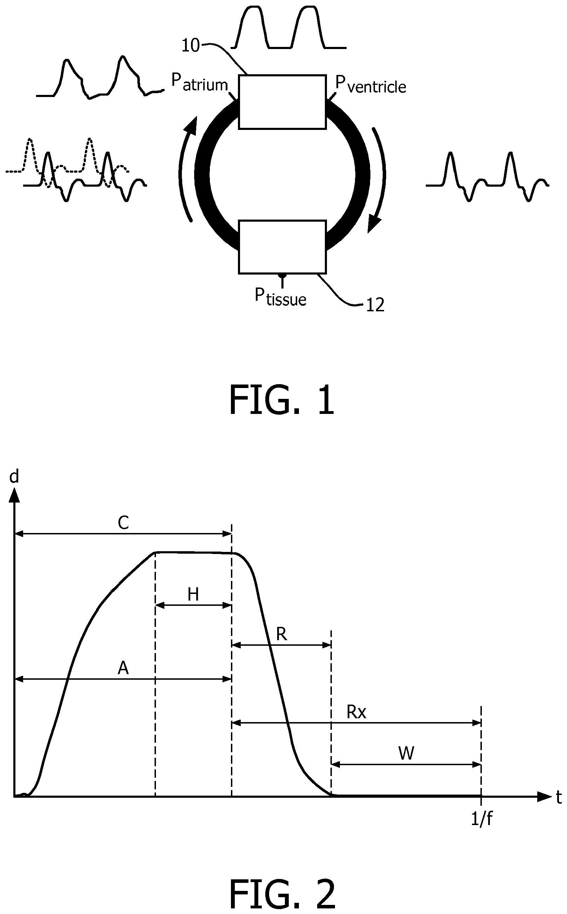

The basic way blood flow is generated using CPR is explained with reference to Figure 1, which provides a simplified view at only one side of the heart. The chest is compressed downwards (compression phase), generating a pressure at the heart 10 shown as both an atrial (Pₐₜᵣᵢᵤₘ) and ventricular (P_{ventricle}) pressure. This pressure is larger than the pressure in the tissue 12 (Pₜᵢₛₛᵤₑ) thereby pushing the blood out of the heart in the tissue and generating a cardiac output and blood flow. This blood flow is proportional to the pressure difference P_{ventricle} - Pₜᵢₛₛᵤₑ. When the chest is released (retraction phase), the pressure at the heart is reduced. Now the pressure in the tissue is higher than the heart pressure and the blood flows back into the heart as the venous return. The return flow is proportional to the pressure difference Pₜᵢₛₛᵤₑ - Pₐₜᵣᵢᵤₘ.

In both the compression and retraction phase, valves in the circulation path prevent the blood flow from circulating the wrong way (however during CPR, these valves are considered not to be 100% functional).

The venous return is delayed with respect to the cardiac output as there is some time needed for the flow/pressure wave to propagate through the arteries, veins and tissue.

At unequal cardiac output and venous return, more blood is pumped out of the heart than that returns to the heart, which leads to shifts of blood out of the heart.

The venous return can for example be impaired by the following two factors.

First, a residual force on the chest may remain during retraction, inducing higher intra thoracic pressure (i.e. higher Pₐₜᵣᵢᵤₘ), thereby lowering the Pₜᵢₛₛᵤₑ - Pₐₜᵣᵢᵤₘ pressure gradient and limiting venous return.

Second, too little time may be provided between compressions, so that the venous return is not complete. This time between compressions (i.e. the wait time) is related to the frequency and duty cycle of the compression waveform. Decreasing wait time beyond the optimum wait time results in diminished venous return and a shift of blood from the thoracic to the abdominal region.

Figure 2 is used to show the different phases of the CPR pressure cycle, and shows the compression depth versus time. The compression period (C) comprises a first application phase (A) of increasing compression depth followed by a hold phase (H). There is then a relaxation period (Rx) comprising a first retraction phase (R) when the pressure is released, and then a wait phase (W). Thus, there are four phases of application, holding, retraction and waiting.

In some animal experiments the venous return was studied. Two examples are shown.

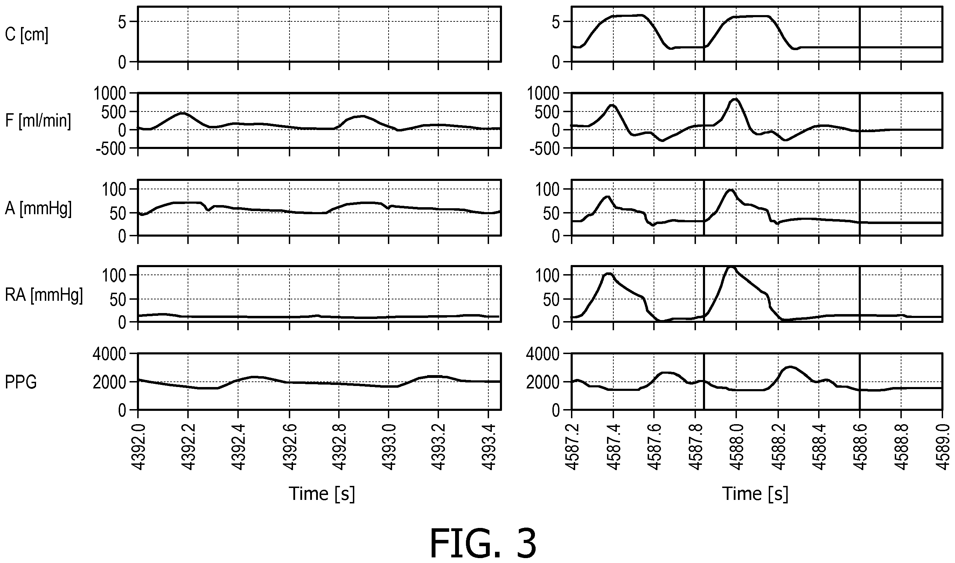

A first example is shown in Figure 3. The left image shows pressure and flow measurements during normal heartbeats and the right image shows the same measurements during CPR.

In order from top to bottom, the plots show the compression depth (C-cm), blood flow (F-ml/min), aortic pressure (A-mmHg), right atrial pressure (RA-mmHg) and a photoplethysmogram (PPG, in arbitrary units).

The PPG signal during both the normal heart beats and during CPR shows its features (e.g. onset and peak) later in time than the pressure and flow, as it is measured peripherally and the pressure/flow signal takes some time to propagate from the heart to the extremities.

During spontaneous heart beats the blood flow shows a first major peak every heartbeat, followed by a second peak (the second peak is for example seen at 4392.4s in Figure 3). During CPR chest compressions, the blood flow waveform shows a first peak after initiation of the chest compression, followed by a retrograde peak (during retraction) and a smaller second positive peak during the wait time. This shows that the flow during CPR is complex and that the flow does not end as soon as the retraction is finished. In this example, the initiation of the second compression occurs during the second flow peak of the first compression as shown by the vertical time lines in the right image. The second compression is fully completed (and this is ensured by not providing a further compression, i.e. there is no compression at 4588.6s).

As the flow of blood after the first compression is not stopped yet, the newly started compression is non optimal, as it pushes on a not completely filled heart. Further, it might block the remaining blood flow back to the heart, thereby limiting venous return.

Other indications of the compressions being done on an incompletely filled heart (at an early point in time where venous return was not completed) are the lower right atrial pressure (12 mmHg at the too early initiated compression versus 17 mmHg at the fully completed compression) and the morphological difference in the PPG signal at the initiation of the too early compression versus the fully completed compression.

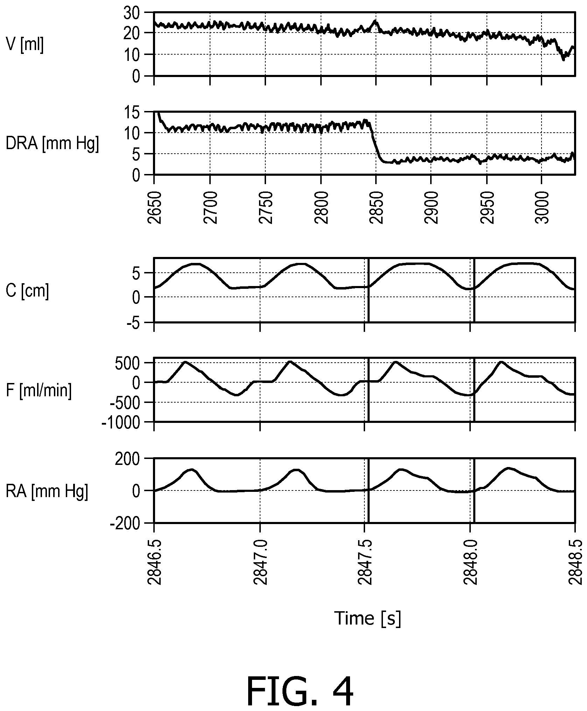

A second example is shown in Figure 4.

The plots show, from top to bottom, the average induced blood volume per compression (V-ml), the per compression end diastolic right atrial pressure (i.e. the right atrial pressure at the start of the next compression, DRA-mmHg), the compression waveform (C-cm), the blood flow (F-ml/min) and the right atrial pressure (RA-mmHg). Note that the DRA signal is the RA signal at the start of each compression.

In this example compressions are given at 120 compression per minute, first 200 seconds at a duty cycle of 40%, followed by 200 seconds at a duty cycle of 60%. Duty cycle is defined by the ratio of the compression period (C) over the complete compression (C+Rx), see Figure 2. The top two plots show the full 400 second period, and the bottom three plots are a zoomed in part at the change of the duty cycle.

At t=2847s, the duty cycle of the compressions is changed from 40 to 60%, resulting in less time between compressions and degradation of venous return, resulting in a lower right atrial filling pressure. This is accompanied by a gradual decrease of per compression blood volume over time. In the lower three rows, the change of duty cycle is zoomed in over 4 compressions. The first 2 compressions have a duty cycle of 40%, the second two a duty cycle of 60%. In the 40% DC compressions, there is enough time for venous return, indicated by the cease of blood flow (i.e. the plateau at 0 ml/s). End diastolic right atrial pressure is 12 mmHg. When going to the higher duty cycle, compressions are started too early, when blood flow is not returned to zero. Also the end diastolic right atrial pressure is decreased (3 mmHg), indicating venous return has not been completed.

CPR devices are known which aim to enhance venous return. These are targeted at increasing the tissue to atrial pressure gradient, and thereby promoting venous return. For example WO96/10984 discloses the use of abdominal compressions between chest compressions to increase tissue pressure and the tissue-arterial gradient. WO 02/091905 discloses decreasing the intra-thoracic pressure between compressions by actively pulling up the chest. US 5 692 498 discloses an approach by which the intra-thoracic pressure is decreased by using an impedance threshold device ("ITD"). This is a valve that is placed at the end of an endotracheal tube. It prevents influx of air into the lung by only opening the valve at a certain pressure gradient. Every compression air is pumped out, thereby reducing the intra-thoracic pressure. During compressions the pressure gradient is not enough to open the valve and let air in. When ventilation is given (with e.g. a bag mask valve device) the pressure gradient becomes larger than the ITD threshold and air flows in. While these approaches enhance the venous return, they don't look for completion of venous return; hence they do not optimize compression frequency. Using these approaches by themselves thus does not guarantee an optimum venous return and optimum compression frequency.

This invention makes use of a physiological measurement of a patient in order to determine when venous return is completed and a new CPR chest compression should be administered, thereby determining the optimal chest compression frequency for a patient.

By timing a chest compression based on the time that venous return is completed (by measurement of the physiological variable), venous return is maximized, so that blood volume shifts from the heart to other organs are minimized, thereby improving the long term circulation of blood instead of only shifting blood. This results in more effective CPR, which can result in higher chance of survival from cardiac arrest.

The compression frequency is optimized for individual patients with this method. Rescuer feedback on compression rate can be given based on a personalized measurement instead of based on a target guideline that is based on a group average.

The physiological measurement is used as a venous return sensor, and the signal is processed to determine the completion of venous return and then trigger a next CPR compression. The output of the system can be used to trigger chest compressions in an automated CPR device in order to automatically tune to the optimal compression frequency of a patient at a certain point in time, or it can be used as feedback signal in an emergency care monitor that gives rescuer feedback on optimal (personalized) compression rate.

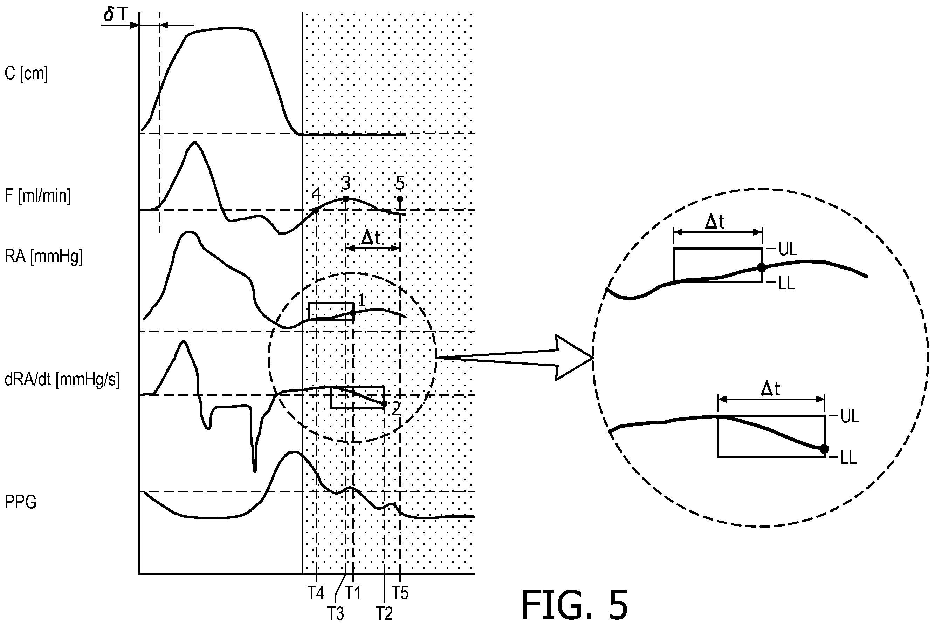

Figure 5 is used to show how some physiological signals can be used to identify the end of the venous flow.

The top plot shows the CPR compression depth (C-cm), the second plot shows the blood flow (F-ml/min), the third plot shows the right atrial pressure (RA-mmHg), the fourth plot shows the derivative with respect to time of the right atrial pressure (dRA/dt - mmHg/s), and the fifth plot show the PPG signal. The x-axis represents time.

A number of possible examples as given below for sensors which can be used to detect the point at which venous return is completed. Examples are then explained of the possible signal processing which can be used.

### Sensor example 1

A blood flow sensor may be used to measure the completion of venous return. This blood flow sensor can for example be an invasive Doppler ultrasound flow sensor or an external ultrasound flow sensor. The flow sensor can be placed over any major artery, preferably over a carotid artery in the neck area.

### Sensor example 2

The right atrial / arterial pressure may be used to determine the completion of venous return. This can be done based on a blood pressure catheter, that ideally is placed near the right atrium, or any other form of blood pressure monitoring system. The use of a blood pressure sensor as a venous return sensor can be justified by the blood flow physiology; as the blood flow is proportional to the blood pressure difference, a constant pressure (or no change of derivative of blood pressure) indicates no changes, hence a zero flow and completion of venous return.

### Sensor example 3

A (photo-) plethysmographic signal (PG) signal may be used to determine the completion of venous return. This can be implemented for example using a PG sensor on the forehead or on an extremity like a finger. The use of a plethysmographic (i.e. volume) sensor as venous return sensor can be justified by the blood flow physiology; as blood volume is the integral of blood flow, a constant PG indicates no flow and a completion of venous return.

### Algorithm example 1

The completion of venous return may be defined as the time at which the physiological signal (blood flow, pressure or PPG) remains between an upper and lower limit (UL and LL in Figure 5) for a certain amount of time. This approach is represented in Figure 5 on the plot of right atrial pressure, and the time point is shown as T1 at the end of the time period Δt.

### Algorithm example 2

The completion of venous return may be defined as the time at which the time derivative of the physiological signal (blood flow, pressure or PPG) remains between an upper and lower limit for a certain amount of time (again UL and LL in Figure 5). This approach is represented in Figure 5 on the plot of the derivative of right atrial pressure, and the time point is shown as T2, at the end of the time period Δt (which may be different to that shown in the right atrial pressure signal).

### Algorithm example 3

The completion of venous return may be defined as the time at which the physiological signal (blood flow, pressure or PG) has its maximum value after the completion of a chest compression. This approach is represented in Figure 5 on the plot of blood flow, and the time point is shown as T3.

### Algorithm example 4

In a blood flow based embodiment, the completion of venous return can be defined as the first time the flow becomes positive after the first large sloshing (negative) peak of blood flow. This approach is represented in Figure 5 on the plot of blood flow, and the time point is shown as T4.

### Algorithm example 5

In all of these embodiments the time point of venous return may incremented by a fixed delay, in order to compensate for a delay between the physical location of the venous return sensor and the actual completion of venous return. This delay is represented in Figure 5 on the plot of blood flow, and the resulting time point is shown as T5. An estimation of this delay can be made by subtracting the time point of the onset of the compression waveform from the time point of the onset of the physiological measurement, for example the time delay δT shown in Figure 5 based on the onset of the flow measurement pulse following the start of the compression cycle. Of course, this requires the compression waveform to be provided as an input to the processor.

This approach can be applied to any sensor signal used by the system. Thus, the delay may generally be obtained by measuring the delay between the start of a CPR compression cycle and the start of the response of the physiological measurement sensor signal to that CPR compression cycle.

Multiple venous return sensors (blood flow, right atrial pressure and PPG) may be combined to form a venous return sensor. Different trigger points may be used in the algorithms explained above (the limit values, the derivative limit values or the maximum signal value) in a single patient depending on the nature of the signal.

The accuracy and robustness of the trigger time may be improved by signal processing techniques, such as averaging of multiple trigger times and predicting trigger times based on previous triggers.

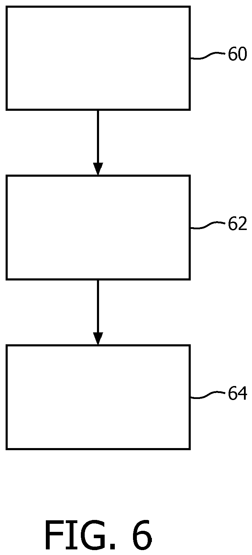

Figure 6 shows a CPR monitoring method. In step 60, a physiological measurement is made to generate a sensor signal which conveys information concerning the point in time at which venous return is completed following a CPR compression. The sensor is one or more of the sensor types discussed above. In step 62 the sensor signal is processed to determine time points at which CPR compression cycles should be administered based on venous return being completed. This step makes use of one or more of the algorithm examples above.

In step 64 output information is provided related to the determined time points.

The output information may be used to control an automated CPR device, and compressions are initiated at the determined time points, thereby optimizing compression frequency for a specific patient at a specific time. The time points may be selected such that a compression is started as soon as possible after venous return is completed in order to have a high as possible blood flow without shifting blood from the heart to other areas. Instead or as well as controlling the CPR frequency, the compression duty cycle may also be varied based on the venous return. For example, the duty cycle may be shortened if venous return is not completed.

The output information may instead be used to provide output information on an emergency care monitor. The information then gives guidance to a caregiver as to when compressions should be given. It may for example give a warning in the case that a compression is started more than a (first) predetermined time before one of the determined time points and/or more than a (second) predetermined time after the determined time point.

Alternatively, the monitor may provide an optimum compression rate value to a caregiver in the form of a frequency at which compressions should be given. This compression rate value is based on venous return information of previous compressions. In either of these ways, the manual compression frequency may be optimized for a specific patient.

In a similar way, the duty cycle can be controlled, for example by providing signals which indicate end of the hold phase. Instead of giving quantitative feedback, qualitative feedback may be signalled to the user in order to reduce the duty cycle. Such qualitative feedback could consist of terms like "retract faster".

Note that the physiological sensor may be combined with other known devices and method which aim to promote venous return, such as impedance threshold devices, active decompression or interposed abdominal compressions.

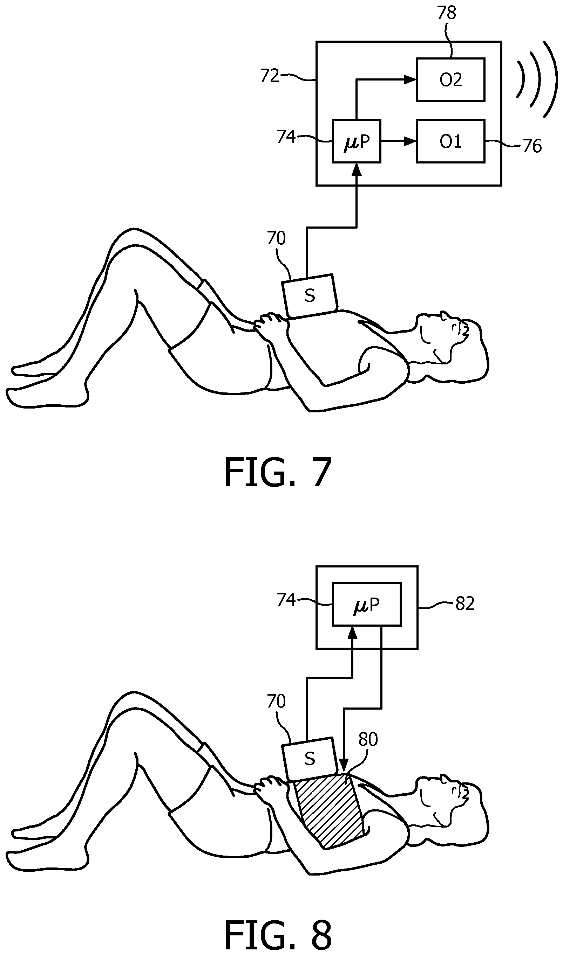

Figure 7 shows the device used as part of a monitor for assisting manual CPR.

The system has a sensor 70 as described above and a processing and output unit 72. The unit 72 has a processor 74 which processes the sensor signals (in the manner explained above) and generates outputs, for example a visual output on a display 76 or LED array, and an audio output from a speaker 78. The sensor may measure the physiological signals used to determine the end of venous return and optionally also the compression cycles themselves, for example to determine time delay information as explained above.

Figure 8 shows the device used as part of an automated CPR device, comprising a chest compression device 80 controlled by a controller 82, which has a processor 74 which processes the signals from the sensor 70. In this case, the processor functions as an output device, providing control signals to the chest compression device 80. The chest compression device may be a belt or a piston based device. In this case, the compression cycle timing is controlled by the system so no additional sensor is needed to derive the compression timing information (to enable time delays to be derived as explained above).

In both cases, the aim is to improve cardiac arrest treatment by taking into account the individual patient response to the CPR.

As outlined above a sensor signal may be selected which conveys information which indicates when the flow returns to zero (or near zero) after a CPR compression cycle. This can be derived directly from a flow measurement or indirectly from another (surrogate) signal, for example a time derivative of pressure, or a time derivative of volume.

As discussed above, a processor is used to analyse the physiological sensor signal. The processor can be implemented in numerous ways, with software and/or hardware, to perform the various functions required. The processor may employ one or more microprocessors that may be programmed using software (e.g., microcode) to perform the required functions. The processor may instead be implemented as a combination of dedicated hardware to perform some functions and a software processor (e.g., one or more programmed microprocessors and associated circuitry) to perform other functions.

Examples of components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform at the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. A cardiopulmonary resuscitation assistance system, comprising:
a sensor (70) configured for making a physiological measurement to generate a sensor signal which conveys information concerning the point in time at which venous return is completed; and
a processor (74) configured for processing the sensor signal to determine time points at which cardiopulmonary resuscitation compression cycles should be administered based on the point in time at which venous return is completed; and
an output device (76,78; 74) configured for providing output information relating to the determined time points.

2. A system as claimed in claim 1, wherein the sensor signal conveys information which indicates when blood flow returns to zero after a cardiopulmonary resuscitation compression cycle.

3. A system as claimed in claim 1 or 2, wherein the sensor (70) comprises a blood flow sensor such as an external ultrasound flow sensor.

4. A system as claimed in claim 3, wherein the processor (74) is adapted to determine the time at which the flow first becomes positive after a negative peak of blood flow.

5. A system as claimed in any preceding claim, wherein the sensor (70) comprises a pressure sensor for measuring the right atrial pressure or a plethysmographic sensor.

6. A system as claimed in any preceding claim, wherein the processor (74) is adapted to determine:
the time at which the physiological measurement sensor signal remains between an upper and lower limit for a certain amount of time or the time at which the derivative of the physiological measurement sensor signal remains between an upper and lower limit for a certain amount of time; or
the time at which the physiological measurement sensor signal has its maximum value after the completion of a chest compression.

7. A system as claimed in any preceding claim, wherein the processor (74) is adapted to apply a delay after the sensed completion of the venous return to compensate for a delay between its physical location of the sensor and the actual completion of venous return.

8. A system as claimed in claim 7, wherein the processor (74) is adapted to determine the delay by measuring the delay between the start of a cardiopulmonary resuscitation compression cycle and the start of the response of the physiological measurement sensor signal to that cardiopulmonary resuscitation compression cycle.

9. An emergency care monitor comprising a cardiopulmonary resuscitation assistance system as claimed in any preceding claim, wherein the processor (74) is adapted to provide:
an output warning signal when a cardiopulmonary resuscitation compression is started more than a predetermined time before and/or after the determined time points at which cardiopulmonary resuscitation compression cycles should be administered; or
an indication of a suitable compression rate corresponding to the determined time points or previously determined time points.

10. An automated cardiopulmonary resuscitation device comprising:
a chest compression system (80); and
a cardiopulmonary resuscitation assistance system as claimed in any one of claims 1 to 8 for controlling the timing of operation of the chest compression system.

## Patentansprüche

1. System zur Unterstützung von Herz-Lungen-Wiederbelebung, umfassend:
einen Sensor (70), der konfiguriert ist zum Vornehmen einer physiologischen Messung, um ein Sensorsignal zu erzeugen, das Informationen hinsichtlich des Zeitpunkts, zu dem venöser Rückfluss abgeschlossen ist, befördert; und
einen Prozessor (74), der konfiguriert ist zum Verarbeiten des Sensorsignals, um Zeitpunkte zu bestimmen, an denen Kompressionszyklen einer Herz-Lungen-Wiederbelebung verabreicht werden sollten, auf der Grundlage des Zeitpunkts, zu dem venöser Rückfluss abgeschlossen ist; und
eine Ausgabevorrichtung (76,78; 74), die konfiguriert ist zum Bereitstellen von Ausgabeinformationen im Zusammenhang mit den bestimmten Zeitpunkten.

2. System nach Anspruch 1, wobei das Sensorsignal Informationen befördert, die anzeigen, wann Blutfluss nach einem Kompressionszyklus einer Herz-Lungen-Wiederbelebung auf null zurückkehrt.

3. System nach Anspruch 1 oder 2, wobei der Sensor (70) einen Blutflusssensor, wie einen externen Ultraschall-Flusssensor umfasst.

4. System nach Anspruch 3, wobei der Prozessor (74) angepasst ist, um die Zeit zu bestimmen, an der der Fluss nach einer negativen Blutflussspitze zum ersten Mal positiv wird.

5. System nach einem der vorstehenden Ansprüche, wobei der Sensor (70) einen Drucksensor zum Messen des rechtsatrialen Drucks oder einen pletyhsmographischen Sensor umfasst.

6. System nach einem der vorstehenden Ansprüche, wobei der Prozessor (74) abgepasst ist zum Bestimmen:
der Zeit, zu der das Sensorsignal der physiologischen Messung eine gewisse Zeitdauer lang zwischen einem oberen und unteren Grenzwert verbleibt, oder der Zeit, zu der die Ableitung des Sensorsignals der physiologischen Messung eine gewisse Zeitdauer lang zwischen einem oberen und unteren Grenzwert verbleibt; oder
der Zeit, zu der das Sensorsignal der physiologischen Messung nach Abschluss einer Brustkompression seinen Höchstwert aufweist.

7. System nach einem der vorstehenden Ansprüche, wobei der Prozessor (74) angepasst ist, um nach dem erfassten Abschluss des venösen Rückflusses eine Verzögerung anzuwenden, um eine Verzögerung zwischen dem physischen Standort des Sensors und dem tatsächlichen Abschluss von venösem Rückfluss zu kompensieren.

8. System nach Anspruch 7, wobei der Prozessor (74) angepasst ist, um die Verzögerung durch Messen der Verzögerung zwischen dem Beginn eines Kompressionszyklus einer Herz-Lungen-Wiederbelebung und dem Beginn der Reaktion des Sensorsignals der physiologischen Messung auf den Kompressionszyklus der Herz-Lungen-Wiederbelebung zu bestimmen.

9. Monitor zur Notfallversorgung, umfassend ein System zur Unterstützung von Herz-Lungen-Wiederbelebung nach einem der vorstehenden Ansprüche, wobei der Prozessor (74) angepasst ist, um bereitzustellen:
eine Warnsignalausgabe, wenn eine Kompression einer Herz-Lungen-Wiederbelebung länger als eine vorbestimmte Zeit vor und/oder nach den bestimmten Zeitpunkten beginnt, an denen Kompressionszyklen einer Herz-Lungen-Wiederbelebung verabreicht werden sollten; oder
einen Hinweis für eine angemessene Kompressionsrate, die den bestimmten Zeitpunkten oder zuvor bestimmten Zeitpunkten entspricht.

10. Automatisierte Vorrichtung für Herz-Lungen-Wiederbelebung, umfassend:
ein Brustkompressionssystem (80); und
ein System zur Unterstützung von Herz-Lungen-Wiederbelebung nach einem der Ansprüche 1 bis 8 zum Steuern der zeitlichen Abstimmung des Betriebs des Brustkompressionssystems.

## Revendications

1. Système d'assistance à la réanimation cardio-respiratoire, comprenant :
un capteur (70) configuré pour établir une mesure physiologique pour générer un signal de capteur qui transporte des informations concernant le point temporel auquel un retour veineux est achevé ; et
un processeur (74) configuré pour traiter le signal de capteur pour déterminer des points temporels auxquels des cycles de compression de réanimation cardio-respiratoire devraient être administrés en se basant sur le point temporel auquel le retour veineux est achevé ; et
un dispositif de sortie (76, 78 ; 74) configuré pour fournir des informations de sortie se rapportant aux points temporels déterminés.

2. Système selon la revendication 1, dans lequel le signal de capteur transporte des informations qui indiquent quand un flux sanguin revient à zéro après un cycle de compression de réanimation cardio-respiratoire.

3. Système selon la revendication 1 ou 2, dans lequel le capteur (70) comprend un capteur de flux sanguin tel qu'un capteur de flux à ultrasons externe.

4. Système selon la revendication 3, dans lequel le processeur (74) est conçu pour déterminer le moment auquel le flux devient d'abord positif après un pic négatif de flux sanguin.

5. Système selon n'importe quelle revendication précédente, dans lequel le capteur (70) comprend un capteur de pression pour mesurer la pression atriale droite ou un capteur pléthysmographique.

6. Système selon n'importe quelle revendication précédente, dans lequel le processeur (74) est conçu pour déterminer :
le moment auquel le signal de capteur de mesure physiologique reste entre une limite supérieure et inférieure pendant un certain temps ou le moment auquel la dérivée du signal de capteur de mesure physiologique reste entre une limite supérieure et inférieure pendant un certain temps ; ou
le moment auquel le signal de capteur de mesure physiologique a sa valeur maximale après l'achèvement d'une compression de poitrine.

7. Système selon n'importe quelle revendication précédente, dans lequel le processeur (74) est conçu pour appliquer un retard après l'achèvement détecté du retour veineux pour compenser un retard entre son emplacement physique du capteur et l'achèvement réel de retour veineux.

8. Système selon la revendication 7, dans lequel le processeur (74) est conçu pour déterminer le retard en mesurant le retard entre le début d'un cycle de compression de réanimation cardio-respiratoire et le début de la réponse du signal de capteur de mesure physiologique à ce cycle de compression de réanimation cardio-respiratoire.

9. Dispositif de contrôle de soins d'urgence comprenant un système d'assistance à la réanimation cardio-respiratoire selon n'importe quelle revendication précédente, dans lequel le processeur (74) est conçu pour fournir :
un signal d'avertissement de sortie quand une compression de réanimation cardio-respiratoire est commencée plus d'un temps prédéterminé avant et/ou après les points temporels déterminés auxquels des cycles de compression de réanimation cardio-respiratoire devraient être administrés ; ou
une indication d'un taux de compression approprié correspondant aux points temporels déterminés ou aux points temporels précédemment déterminés.

10. Dispositif de réanimation cardio-respiratoire automatisé comprenant :
un système de compression de poitrine (80) ; et
un système d'assistance à la réanimation cardio-respiratoire selon l'une quelconque des revendications 1 à 8 pour commander la synchronisation de fonctionnement du système de compression de poitrine.
